Europäisches Patentamt

European Patent Office

Office européen des brevets

(19)

(11) Veröffentlichungsnummer: **0 269 849 B1**

# EUROPÄISCHE PATENTSCHRIFT

(12)

(45) Veröffentlichungstag der Patentschrift: **12.08.92**

(51) Int. Cl.⁵: **A61B 5/0235**

(21) Anmeldenummer: **87115686.5**

(22) Anmeldetag: **26.10.87**

(54) **Ablassventil für Blutdruckmessgeräte.**

(30) Priorität: **03.11.86 DE 3637271**

(43) Veröffentlichungstag der Anmeldung:
**08.06.88 Patentblatt 88/23**

(45) Bekanntmachung des Hinweises auf die
Patenterteilung:
**12.08.92 Patentblatt 92/33**

(84) Benannte Vertragsstaaten:
**AT BE CH DE ES FR GB GR IT LI NL SE**

(56) Entgegenhaltungen:
**DE-B- 2 429 046
DE-U- 8 629 328
GB-A- 2 125 148
US-A- 4 200 259**

(73) Patentinhaber: **Speidel + Keller GmbH + Co. KG
Zollernstrasse 2
W-7455 Jungingen(DE)**

(72) Erfinder: **Speidel, Blasius
Hochmeisterstrasse 33
W-7455 Jungingen(DE)**

(74) Vertreter: **Kastner, Hermann, Dipl.-Ing.
Osterholzallee 89
W-7140 Ludwigsburg(DE)**

# Beschreibung

Blutdruckmeßgeräte weisen ein Drucksystem mit einer aufpumpbaren Manschette, mit einem Druckerzeuger, mit einem Druckmeßgerät und mit einem Ablaßventil zum Verringern des Überdruckes in dem Drucksystem auf.

Bei einem bekannten Ablaßventil ist in einem Ventilgehäuse eine Auslaßleitung vorhanden, die ins Freie geht. Im Zuge der Auslaßleitung ist ein Ventilsitz vorhanden, der durch eine hohle Kegelstumpffläche gebildet wird, die die Auslaßleitung ringförmig umgibt. Mit diesem Ventilsitz wirkt ein kegeliger Ventilkörper zusammen, der in der Längsachse des Ventilsitzes relativ zum Ventilsitz hin und her bewegbar geführt ist. Zu diesem Zweck ist der Ventilkörper mit einer Schraubkappe verbunden, die mittels eines Gewindes mittig zur Längsachse des Ventilsitzes und damit des Ventilkörpers verstellbar geführt ist. Zur Verschließen des Drucksystems beim Aufpumpen wird der Ventilkörper mittels der Schraubkappe durch eine Schraubbewegung gegen den Ventilsitz gedrückt. Dadurch, daß durch die Gewindeverbindung zwischen der Schraubkappe und dem Ventilgehäuse eine beträchtliche Kraftübersetzung stattfindet, darf der Kegelgesamtwinkel des Ventilsitzes und des Ventilkörpers nicht zu klein sein, um ein Festklemmen des Ventilkörpers infolge der Reibung zwischen den beiden Kegelflächen zu verhindern. Aus diesem Grunde beträgt der Kegelgesamtwinkel im allgemeinen 60°. Dennoch tritt zwischen den beiden Kegelflächen bereits eine deutlich fühlbare Hafttreibung ein, wenn für einen sicheren Verschluß des Ablaßventils die Schraubkappe mit einer gewissen Kraft angezogen wird.

Aus GB-A-2 125 140 ist ein Ablaßventil gemäß Oberbegriff des Anspruchs 1 bekannt.

Infolge des verhältnismäßig stumpfen Kegelwinkels der beiden Dichtflächen tritt schon bei einer sehr geringen Axialbewegung des Ventilkörpers gegenüber Ventilsitz eine verhältnismäßig große Querschnittsveränderung des dann sich bildenden Durchlaßquerschnittes des Ablaßventils ein. Da beim Beginn der Blutdruckmeßung der Innendruck im Drucksystem einen Höchstwert hat, tritt beim Öffnen des Ablaßventils eine verhältnismäßig hohe Auströmgeschwindigkeit auf, die wegen des begrenzten Volumens des Drucksystems zu einem entsprechend schnellen Druckablaß führt. Dieser schnelle Druckablaß erschwert die zuverläßige Erfassung des systolischen Blutdruckwertes, der vom Druckmeßgerät abgelesen werden muß, sobald das erste Korotkoff'sche Strömungsgeräusch in der Arterie unterhalb derjenigen Stelle auftritt, die durch die Manschette bei dem zuvor herrschenden höhern Überdruck abgeschnürt war. Die Feinfühligkeit des Druckablaßes gerade zu Beginn des Meßvorganges wird zusätzlich dadurch erschwert, daß infolge des zuvor geschilderten Reibschlußes zwischen den beiden Dichtflächen die Schraubkappe mit einem nicht unerheblichen Anfangsmoment gedreht werden muß, bis der höhere Haftreibwert zwischen den Dichtungsflächen überwunden ist und der geringere Gleitreibwert erreicht wird. Da dieser Übergang von der Haftreibung zur Gleitreibung schlagartig auftritt, führt das durch die Finger aufgebrachte höhere Anfangsmoment dazu, daß die Schraubkappe über den Drehweg hinaus bewegt wird, der für einen feinfühligen Druckablaß erforderlich wäre. Nach diesem Anfangsruck muß daher die Schraubkappe mit dem Ventilkörper zunächst wieder in schließendem Sinne etwas zurückbewegt werden, um die gewünschte Ablaßgeschwindigkeit für die aus dem Drucksystem entweichende Luft zu erreichen.

Bei einem anderen bekannten Blutdruckmeßgerät (DE-OS 21 18 295) ist die Feinfühligkeit des Druckablaßes auch im Anfangsstadium der Blutdruckmessung dadurch möglich, daß ein verhältnismäßig schlanker Ventilkegel benutzt wird. Die Betätigung dieses Ventilkegels erfolgt mittels einer Schwenktaste, um den durch dem schlanken Kegelwinkel erforderlichen größeren Verstellweg zu erreichen. Wenngleich die Betätigung des Ablaßventils mittels der Schwenktaste wesentlich bequemer ist und wegen des günstigeren Ablaßverhaltens dieses Ventils eine sehr feinfühlige Druckreduzierung möglich ist, gibt es immer noch Personen, die aus beruflichen Gründen oder aus sonstigen Gründen öfter ein Blutdruckmeßgerät benutzen und die sich von dem ihnen aus früherer Zeit bekannten Blutdruckmeßgerät mit der Schraubkappe nicht auf das andere Ablaßventil mit der Schwenktaste umstellen wollen.

Der Erfindung liegt die Aufgabe zugrunde, das bekannte Ablaßventil mit der Schraubkappe so umzugestalten, daß damit eine Druckreduzierung mit einer höheren Feinfühligkeit als bisher möglich wird.

Diese Aufgabe wird durch ein Ablaßventil mit den im Anspruch 1 angegebenen Merkmalen gelöst.

Dadurch, daß der Ventilkörper als schlanker Kegelstumpf ausgebildet ist, ist bei einer axialen Betätigung des Ventilkörpers die Änderung der Fläche der Durchlaßöffnung zwischen dem Ventilkörper und dem Ventilsitz nur verhältnismäßig gering, so daß insbesondere in dem an die Schließstellung anschließenden Anfangsbereich des Öffnungshubes des Ventilkörpers eine sehr feinfühlige Dosierung des Luftaustrittes möglich ist, der eine entsprechend feinfühlige Reduzierung des Druckes im Drucksystem ergibt. Dadurch, daß das Gewinde für die axiale Verstellbewegung der Schraubkappe als zweigängiges Steilgewinde ausgebildet ist, wird trotz dem schlanken Kegel der Betätigungsweg der Schraubkappe in Umfangsrichtung auf ein solches Maß verkleinert, daß eine bequeme Betätigung der Schraubkappe mittels zweier Finger ohne ein- oder mehrmaliges Umsetzen möglich ist, und zwar ohne daß dabei die Feinfühligkeit des Luftablasses vermindert wäre. Dadurch, daß der Ventilkörper auf der Innenseite des Ventilsitzes, d.h. auf der dem Drucksystem zugekehrten Seite des Ventilsitzes, angeordnet ist und durch eine Schließfeder in schließendem Sinne am Ventilsitz festgehalten wird, wird erreicht, daß das Ablaßventil in Abwesenheit einer Öffnungskraft selbsttätig schließt, und zwar lediglich unter der Gasdruckkraft, die sich aus dem Überdruck im Drucksystem und aus der Querschnittsfläche des Ventilkörpers am Ventilsitz ergibt, sowie allenfalls noch unter der verhältnismäßig geringen zusätzlichen Kraft der Schließfeder. Dadurch, daß mittels der Schraubkappe der Ventilkörper lediglich in öffnendem Sinne aus der Schließstellung heraus vom Ventilsitz abgehoben werden kann und er im umgekehrten Sinne nicht in den Ventilsitz hineingedrückt oder hineingezogen werden kann, wird vermieden, daß der Ventilkörper sich infolge seiner schlanken Ausbildung mit kleinem Kegelgesamtwinkel im Ventilsitz so fest verkeilt, daß er nur unter Anwendung erheblicher Betätigungskräfte wieder daraus herausbewegt werden kann. Die erforderliche Öffnungskraft für den Ventilkegel bleibt dadurch stets gleich groß und ist insgesamt nur verhältnismäßig gering. Dadurch kann das bei dem bekannten Ablaßventil mit außenliegendem Ventilkörper häufig auftretende Festsitzen des Ventilkörpers im Ventilsitz gar nicht auftreten, weil in diesem Sinne eine Fehlbetätigung des Ablaßventils nicht möglich ist. Dadurch daß die Schraubkappe mit dem Ventilkörper nur über ein Stützlager gekoppelt ist, erfährt der Ventilkörper keine Drehbewegung bei einer drehenden Betätigung der Schraubkappe sowohl bei einer Auswärtsbewegung des Ventilkörpers bis zum Aufsitzen an Ventilsitz, wie auch bei einer Einwärtsbewegung beim Abheben von Ventilsitz.

Durch eine Ausgestaltung des Ablaßventils nach Anspruch 2 wird einerseits eine sehr große Feinfühligkeit bei der Druckreduzierung und andererseits eine große Leichtgängigkeit gewährleistet. Bei einer Ausgestaltung des Ablaßventils nach Anspruch 3 wird gerade noch Selbsthemmung der Schraubkappe erreicht, so daß die auf den Ventilkörper von innen her wirkende Gasdruckkraft und die Schließkraft der Schließfeder die Schraubkappe nicht auswärts bewegen können. Dadurch wird eine einmal gewählte Einstellung des Ventilkörpers auch dann engehalten, wenn die Schraubkappenicht festgehalten wird. Dadurch wird die Bedienungsperson des Blutdruckmeßgerätes davon eintlastet, die Einhaltung der Ablaßgeschwindigkeit des Überdruckes überwachen zu müssen. Durch eine Ausgestaltung des Ablaßventils nach Anspruch 4 wird eine sehr leichte Drehbarkeit der Schraubkappe gegenüber dem Ventilkörper erreicht. Bei einer Ausgestaltung des Ablaßventils nach Anspruch 5 wird ein Schnellablaß ermöglicht, indem nach der Ermittlung des unteren Blutdruckwertes mit einer verhältnismäßig geringen zusätzlichen Drehbewegung der Schraubkappe der Ventilkörper in diejenige Axialstellung gebracht wird, in der der Bypass wirksam wird. Danach wird durch den erheblich vergrößerten Durchlaßquerschnitt der restliche Überdruck im Drucksystem in kürzester Zeit abgebaut, so daß die Meßmanschette schon sehr kurze Zeit nach Beendigung der eigentlichen Messung so weit entlüftet und entspannt ist, daß sie abgenommen werden kann. Das verkürzt die Einwirkungszeit der Manschette auf die Untersuchungsperson ganz erheblich, denn der Luftdruck nähert sich bei normalem Außlaßquerschnitt nur asymptotisch dem Nullpunkt, wodurch die Zeitspanne sehr lange ist, bis die Manschette nach ausreichender Druckentlastung abgenommen werden kann. Daneben wird auch die Bedienungsperson des Blutdruckmeßgerätes entlastet, da sie schon sehr bald nach Abschluß des eigentlichen Meßvorganges die Manschette abnehmen kann und sich beispielsweise der Aufzeichnung der Blutdruckwerte oder weiteren Untersuchungen der Untersuchungsperson zuwenden kann.

Im folgenden wird die Erfindung anhand zweier in der Zeichnung dargestellter Ausführungsbeispiele näher erläutert. Es zeigen:

Fig. 1    einen Längsschnitt eines ersten Ausführungsbeispieles des Ablaßventils;

Fig. 2    eine teilweise geschnitten dargestellte Ansicht eines abgewandelten Ausführungsbeispieles des Ablaßventils.

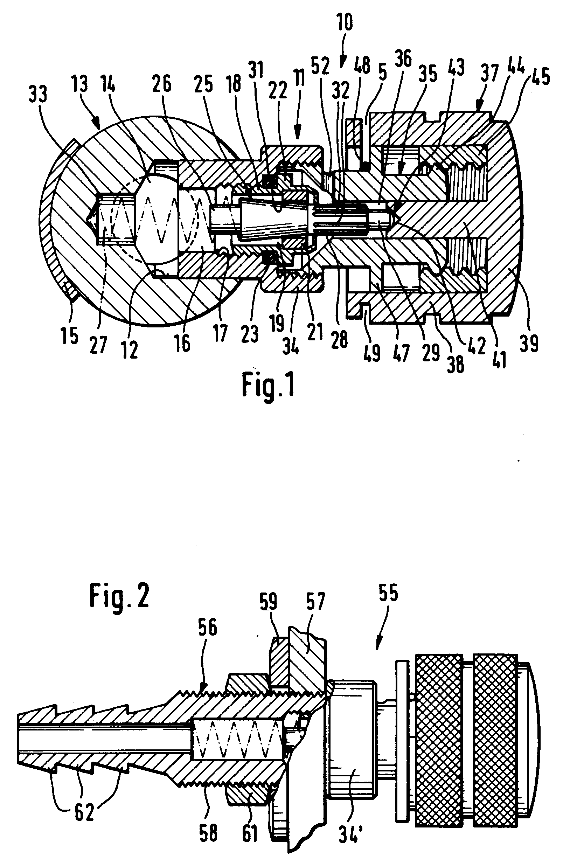

Das aus Fig. 1 ersichtliche Ablaßventil 10 weist einen Ventilkörper 11 auf, der in einer Querbohrung 12 eines zylindrischen Grundgehäuses 13 des im übrigen nicht dargestellten Blutdruckmeßgerätes sitzt. Dieses Grundgehäuse 13 weist ein axiale Durchgangsöffnung 14 auf, an die am einen Ende des Grundgehäuses ein Druckerzeuger, etwa in Form einer Gummiballpumpe mit einem selbsttätigen Einlaßventil angeschlossen ist, und an dessen anderem Ende eine Verbindungsleitung zu einer Meßmanschette und zu einem Druckmeßgerät des Blutdruckmeßgerätes angeschlossen ist, die alle zusammen das Blutdruckmeßgerät insgesamt bilden. An der Seite des Druckgehäuses 13 ist ein sogenannter Löffel 15 angedeutet, der parallel zum Gummiball der Gummiballpumpe verläuft und das Betätigen der Gummiballpumpe erleichtert.

Der Ventilkörper 11 ist als zylindrischer Körper ausgebildet, der gasdicht in der Querbohrung sitzt, in die er im allgemeinen eingelötet ist. Er kann darin aber auch mittels eines Gewindes mit oder ohne zusätzliche Dichtungsmittel eingeschraubt sein. Im Inneren des Ventilkörpers 11 ist eine mittig angeordnete Auslaßleitung 16 vorhanden, die ebenso wie die Durchgangsöffnung 14 einen Teil des Drucksystems des Blutdruckmeßgerätes bildet. Im Bereich der Auslaßleitung 16 ist am Ventilkörper 11 ein Innengewinde 17 vorhanden, in das ein zylindrischer und mit Schraubengewinde versehener Ventilsitzträger 18 eingeschraubt ist. Dieser weist eine axiale Durchgangsöffnung 19 auf, die in dem nach außen gekehrten Endbereich einen Absatz aufweist, in den ein Ventilsitzring 21 eingesetzt ist, der durch eine dünnwandige axiale Verlängerung des Ventilsitzträgers 18 dadurch festgehalten wird, daß diese Verlängerung nach dem Einsetzen des Ventilsitzringes 21 nach innen geringfügig umgebördelt wird. Auf der Außenseite weist der Ventilsitzträger 18 einen Bund 22 auf, durch den ein Dichtunsring 23 an einer umlaufenden Ausnehmung des Ventilkörpers 11 zwecks Abdichtung angepreßt wird.

Mittig zum Ventilsitzring 21 ist ein Ventilkörper 25 angeordnet. Er ist als schlanker Kegelstumpf ausgebildet, dessen Kegelgesamtwinkel 6° beträgt. Der Ventilkörper 25 ist ein Kunststoff-Formkörper aus Polyamid. An seinem nach innen gekehrten Ende mit dem größeren Kegeldurchmesser ist ein Führungszapfen 26 für eine Schließfeder 27 angeformt. An dem außen gelegenen Ende des Kegelstumpfes mit dem kleineren Kegeldurchmesser schließt ein näherungsweise zylindrischer Fortsatz 28 an, an dessen von Kegelstumpf abgekehrten Ende ein im Durchmesser abgesetzter Lagerzapfen 29 anschließt.

Der Ventilsitzring 21 ist als Kreisringzylinder ausgebildet, dessen dem Inneren des Grundgehäuses 13 zugekehrte innere Ringkante den Ventilsitz 31 bildet. Der Ventilsitzring 21 ist aus Polytetrafluoräthylen hergestellt, das neben einer guten Gleiteigenschaft auch eine gewisse Elastizität hat, durch die die Ringkante bei der Anlage des Ventilkörpers elastisch so weit nachgibt, daß sich eine kleine kegelstumpfförmige Anlagefläche als eigentliche Dichtfläche bildet.

Am Ventilkörper 25 beginnt der zylindrische Fortsaz 28 an einer Stelle, die in der Schließstellung des Ventilkörpers 25 (Fig. 1) vom Ventilsitz 31 etwas weiter entfernt ist, als es dem normalen Öffnungshub des Ventilkörpers entspricht. Auf der Außenseite des Fortsatzes 28 ist eine Anzahl Ausnehmungen 32 vorhanden, die am Umfang des Fortsatzes 28 gleichmäßig verteilt sind und die einen etwa dreieckförmigen Querschnitt haben. Die Ausnehmungen 32 sind axial ausgerichtet und münden frei am Ende des Fortsatzes 28. Die Summe der Querschnittsflächen der Ausnehmungen 32 bildet einen zusätzlichen Auslaßquerschnitt zu der ringförmigen Querschnittsfläche zwischen dem in eine gewisse Offenstellung bewegten Ventilkörper 25 und dem Ventilsitz 31. Dieser zusätzliche Auslaßquerschnitt tritt innerhalb eines verhältnismäßig kleinen Abschnittes des Bewegungsweges des Ventilkörpers zu der normalen Querschnittsfläche hinzu und wirkt dadurch als Schnellablaß für die Druckluft im Drucksystem des Blutdruckmeßgerätes.

In der Fluchtlinie des Ventilgehäuses 11 ist im Grundgehäuse 13 eine kreiszylindrische Einsenkung 33 vorhanden, in die das eine Ende der Schließfeder 27 eingesetzt ist. Sie wird darin durch die Umfangswand in radialer Richtung geführt und durch die Stirnfläche in axialer Richtung abgestützt. Das andere Ende der Schließfeder wird vom Führungszapfen 26 in radialer Richtung geführt, der gegenüber dem großen Durchmesser der Kegelstumpfform des Ventilkörpers 25 abgesetzt ist. An dessen Stirnfläche stützt die Schließfeder 27 sich in axialer Richtung am Ventilkörper 25 ab.

An das Ventilgehäuse 11 schließt ein kreisringförmiger Kragen 34 an. Seine lichte Weite ist größer als der Außendurchmesser des Bundes 22 am Ventilsitzträger 18. Die Innenwand des Kragens 34 ist mit einem Innengewinde versehen. Darin ist ein Führungskörper 35 eingeschraubt. Er ist weitgehend zylindrisch ausgebildet. Er weist mittig zu seiner Längsachse, die mit der Längsachse des Ventilgehäuses 11 fluchtet, ein kreiszylindrisches Führungsloch 36 auf. In dieses Führungsloch 36 ragt der Fortsatz 28 teilweise hinein. Das Führungsloch 36 dient der Führung einer Schraubkappe 37. Diese weist einen Mantel 38 auf, der an einem Ende durch einen Boden 39 verschlossen ist. Der Boden 39 ist auf seiner Außenseite leicht gewölbt. Auf seiner Innenseite ist mittig zur Längsachse der Schraubkappe 37 ein Führungszapfen 41 vorhanden, der einstückig mit dem Boden 39 und dem Mantel 38 ausgebildet ist. Der Führungszapfen 41 ist auf das Führungsloch so abgestimmt, daß sich zwischen den beiden Teilen ein Laufsitz ergibt.

Die dem Ventilgehäuse 11 zugekehrte Stirnseite des Führungszapfens 41 ist als hohlkegelige Lagerpfanne 42 ausgebildet, an der sich die endseitige Ringkante des Lagerzapfens 29 des Ventilkörpers 25 in axialer Richtung abstützt, wobei der Lagerzapfen 29 zugleich in radialer Richtung geführt wird. Diese Ringkante kann leicht angefast oder im Querschnitt leicht gerundet sein. Die Lagerpfanne 42 und der Lagerzapfen 29 bilden zusammen ein Stützlager 43 für den Ventilkörper 25.

An dem vom Ventilgehäuse 11 abgekehrten Endbereich des Führungskörpers 35 ist ein Gewindeteil 44 vorhanden, der durch einen Bund gebildet wird, der mit einem Außengewinde versehen ist. Auf der Innenseite des Mantel 38 der Schraubkappe 37 ist ein Gewindefutter 45 in Form eines Kreisringzylinders fest eingesetzt. Diese Gewindefutter 45 ist mit einem Innengewinde versehen, das auf das Außengewinde des Gewindeteils 44 abgestimmt ist.

Die Schraubkappe 37 ist in herkömmlicher Weise mit einer Ausdrehsicherung 46 versehen. Diese weist einen mit dem Führungskörper 35 einstückig gefertigten Bund 47 und einen Federbügel 48 auf. Der Bund 47 hat einen Außendurchmesser, der nur wenig kleiner als der Innendurchmesser des Mantels 38 ist. Der Federbügel 48 ist in eine Ringnut 49 auf der Außenseite des Mantels 38 eingelegt, in der er sich durch seine federnden Enden selbst festhält. Auf einer Seite ist der Mantel 38 mit einem bis zur Innenseite reichenden Schlitz 51 versehen, durch den sich ein gerader Längenabschnitt des Federbügels 48 hindurch erstreckt. Der Bund 47 ist am Führungskörper 35 in derjenigen Ebene angeordnet, die der Federbügels 48 gerade dann erreicht, wenn die Schraubkappe 37 so weit vom Ventilgehäuse 11 weggeschraubt wurde, daß einerseits der Ventilkörper 25 sicher am Ventilsitz 31 anliegt und andererseits der Lagerzapfen 29 noch nicht aus der Lagerpfanne 42 ausgetreten ist.

Am Führungskörper 35 ist in axialer Richtung außerhalb des Ventilsitzträgers 18 eine seitliche Auslaßöffnung 52 vorhanden, durch die die zwischen dem Ventilsitz 31 und dem mehr oder weniger weit davon abgehobenen Ventilkörper 25 ausströmende Luft ins Freie entweichen kann.

Das aus Fig. 2 ersichtliche Ablaßventil 55 ist gegenüber dem Ablaßventil 10 lediglich hinsichtlich der Verbindung mit dem Blutdruckmeßgerät abgewandelt. Sein Ventilgehäuse 56 ist an einer Wand 57 des im übrigen nicht dargestellten Gehäuses des anders gestalteten Blutdruckmeßgerätes angeschraubt. Dafür ist das Ventilgehäuse 56 mit einem Außengewinde 58 versehen. Das Ventilgehäuse 56 ist durch ein Loch in der Wand 57 so weit hindurchgesteckt, daß sein Kragen 34′ an der Außenseite der Wand 57 anliegt. Von der Innenseite des Gehäuses her ist eine Unterlegscheibe 59 aufgesteckt und eine Mutter 61 aufgeschraubt. Damit ist das Ablaßventil 55 mit dem Gehäuse fest verbunden. Im Anschluß an das Außengewinde 58 ist das Ventilgehäuse 56 als Schlauchtülle 62 ausgebildet, auf die ein Verbindungsschlauch aufgesteckt wird, über den das Ablaßventil 55 mit den übrigen Teilen des Drucksystems verbunden wird.

**Patentansprüche**

1.  Ablaßventil für Blutdruckmeßgeräte mit den Merkmalen:
    - in einem Ventilgehäuse ist eine ins Freie mündende Auslaßleitung vorhanden,
    - in der Auslaßleitung ist ein ringförmiger Ventilsitz angeordnet,
    - es ist ein kegeliger Ventilkörper vorhanden, der zumindest annähernd in der Längsachse des Ventilsitzes relativ zu diesem bewegbar ist,
    - für die Betätigung des Ventilkörpers ist eine Schraubkappe vorhanden, die in der Längsachse des Ventilkörpers mittels eines Gewindes axial verstellbar ist, dessen einer Gewindeteil mit der Schraubkappe und dessen anderer Gewindeteil mit dem Ventilgehäuse verbunden ist,
    **gekennzeichnet** durch die Merkmale:
    - der Ventilsitz (31) ist dem Innenraum (14) des Ventilgehäuses (11) zugekehrt,
    - der Ventilkörper (25) ist als schlanker Kegelstumpf ausgebildet, dessen großer Durchmesser dem Innenruam (14) des Ventilgehäuses (11) zugekehrt ist,
    - im Ventilgehäuse (11) ist eine Schließfeder (27) angeordnet, die sich einerseits am Ventilgehäuse (11; 13) abstützt und die andererseits auf den Ventilkörper (25) eine Schließkraft in Richtung auf den Ventilsitz (31) hin ausübt,
    - die Schraubkappe (37) weist ein Stützlager (43) für den Ventilkörper (25) auf, an den das nach außen gekehrte Ende (29) des Ventilkörpers (25) relativ zur Schraubkappe (37) um seine Längsachse drehbar gelagert und zugleich axial abgestützt ist,
    - das Gewinde für die axiale Verstellbewegung der Schraubkappe (37) ist als zweigängiges Steilgewinde ausgebildet.

2.  Ablaßventil nach Anspruch 1
    **gekennzeichnet** durch die Merkmale:
    - der Ventilkörper (25) hat einen Kegelgesamtwinkel von zumindest annähernd 6°,
    - der Ventilsitz (31) ist bevorzugt als Ringkante eines hohlzylindrischen Ventilsitzringes (21) ausgebildet, die vorzugsweise leicht angefast ist.

3.  Ablaßventil nach Anspruch 1 oder 2
    **gekennzeichnet** durch das Merkmal:
    - der mittlere Steigungswinkel des Steilgewindes ist höchstens gleich, vorzugsweise geringfügig kleiner als der Reibungswinkel ($\mu$) der Werkstoffpaarung der beiden Gewindeteile (44; 45) ausgebildet.

4.  Ablaßventil nach einem der Ansprüche 1 bis 3
    **gekennzeichnet** durch die Merkmale:
    - das Stützlager (43) wird durch eine hohlkegelige Lagerpfanne (42) und durch eine Ringkante eines Lagerzapfens (29) gebildet, die vorzugsweise angefast oder im Querschnitt abgerundet ist,

- vorzugsweise ist die Lagerpfanne (42) an der Schraubkappe (37) und der Lagerzapfen (29) am Ventilkörper (25) angeordnet, wobei der Lagerzapfen (29) gegenüber dem Ventilkörper (25) auf einen kleineren Durchmesser abgesetzt ist.

5. Ablaßventil nach einem der Anprüche 1 bis 4 **gekennzeichnet** durch die Merkmale:
- der Ventilkörper (25) ist mit einem Bypass (32) zu seiner Dichtfläche versehen, der sich vom drucklosen Bereich des Ventilkörpers (25) aus in Richtung auf den druckbeaufschlagten Bereich bis zu einer Stelle seiner Dichtfläche erstreckt, die bei normalem Öffnungshub des Ventilkörpers (25) noch im Bereich der Drosselwirkung des Ventilsitzes (31) gelegen ist,
- vorzugsweise wird der Bypass durch eine oder mehrere Ausnehmungen (32) gebildet, die, bei mehreren Ausnehmungen, gleichmäßig am Umfang verteilt angeordnet sind und vorzugsweise axial ausgerichtet sind.

## Claims

1. An outlet valve for blood pressure measuring apparatus with the features:
    - a discharge line issuing to the outside is present in a valve housing,
    - an annular valve seat is arranged in the discharge line,
    - there is a tapered valve body which-at least approximately in the longitudinal axis of the valve seat-is movable relative to the latter,
    - there is a screw cap for operation of the valve body, this screw cap being axially displaceable in the longitudinal axis of the valve body by means of a thread whose one threaded portion is connected to the screw cap and whose other threaded portion is connected to the valve housing,
    characterized by the features:
    - the valve seat (31) is facing the interior (14) of the valve housing (11),
    - the valve body (25) is constructed as a slender truncated cone whose large diameter is facing the interior (14) of the valve housing (11),
    - in the valve housing (11) there is arranged a closing spring (27) which on the one hand is supported on the valve housing (11; 13) and on the other hand exercises on the valve body (25) a closing force in the direction of the valve seat (31),
    - the screw cap (37) has a support bearing (43) which is for the valve body (25) and on which the outward-directed end (29) of the valve body (25) is both axially supported and mounted so as to be rotatable relative to the screw cap (37) about its longitudinal axis,
    - the thread for the axial displacement movement of the screw cap (37) is constructed as a two-start coarse thread.

2. An outlet valve in accordance with Claim 1, characterized by the features:
    - the valve body (25) has a taper total angle of at least approximately 6°,
    - the valve seat (31) is preferably constructed as an annular edge of a hollow-cylindrical valve seat ring (21), this annular edge preferably being slightly chamfered.

3. An outlet valve in accordance with Claim 1 or 2, characterized by the feature:
    - the mean angle of inclination of the coarse thread is constructed to be at most equal, preferably slightly smaller, than the angle of friction ($\mu$) of the material mating of the two threaded portions (44; 45).

4. An outlet valve in accordance with any one of Claims 1 to 3, characterized by the features:
    - the support bearing (43) is formed by a hollow-conical bearing socket (42) and by an annular edge of a bearing pin (29), which annular edge is preferably chamfered or has a rounded-off cross-section,
    - the bearing socket (42) is preferably arranged on the screw cap (37) and the bearing pin (29) preferably on the valve body (25), the bearing pin (29) forming an offset relative to the valve body (25) due to the smaller diameter of the former.

5. An outlet valve in accordance with any one of Claims 1 to 4, characterized by the features:
    - the valve body (25) is provided with a by-pass (32) to its sealing surface, this by-pass extending from the pressure-free region of the valve body (25) in the direction of the pressured region up to a site of its sealing surface which with a normal opening lift of the valve body (25) is still located in the region of the throttling effect of the valve seat (31),

- the by-pass is preferably formed by one or a plurality of recesses (32) which in the case of a plurality of recesses are arranged distributed uniformly on the circumference and are preferably axially directed.

**Revendications**

1. Soupape de purge pour des appareils de mesure de la pression sanguine, soupape dans laquelle:

   - une canalisation de purge débouchant à l'air libre est prévue dans un boîtier de soupape,
   - dans cette canalisation de purge est disposé un siège de soupape de forme annulaire,
   - il est prévu un corps de soupape conique qui est susceptible d'être déplacé, tout au moins approximativement selon l'axe longitudinal du siège de soupape par rapport à celui-ci,
   - pour l'actionnement du corps de soupape, il est prévu un bouchon fileté, qui est susceptible d'être déplacé axialement selon l'axe longitudinal du corps de soupape au moyen d'un filetage, dont une partie est reliée au bouchon fileté et l'autre au boîtier de soupape,
   
   soupape de purge caractérisée en ce que :
   - le siège de soupape (31) est tourné vers l'espace interne (14) du carter de soupape (11),
   - le corps de soupape (25) est réalisé sous la forme d'un cône tronqué élancé, dont le plus grand diamètre est tourné vers l'espace interne (14) du boîtier de soupape (11),
   - dans le boîtier de soupape (11) est disposé un ressort de fermeture (27) qui prend appui, d'une part, contre le boîtier de soupape (11; 13) et qui, d'autre part, exerce sur le corps de soupape (25) un effort de fermeture en direction du siège de soupape (31),
   - le bouchon fileté (37) comporte pour le corps de soupape (25) un palier d'appui (43), contre lequel l'extrémité (29), allant vers l'extérieur, du corps de soupape (25) est montée de façon à pouvoir tourner autour de son axe longitudinal par rapport au bouchon fileté (37), et elle prend en même temps appui axialement contre ce palier,
   - le filetage pour le déplacement axial du bouchon fileté (37) est réalisé sous la forme d'un filetage rapide à deux filets,

2. Soupape de purge selon la revendication 1, caractérisée en ce que :
   - le corps de soupape (25) a un angle total de cône d'au moins approximativement 6°,
   - le siège de soupape (31) est, de préférence, réalisé sous la forme d'une arête annulaire d'un anneau de siège de soupape (21) cylindrique creux qui est, de préférence, légèrement chanfreinée.

3. soupape de purge selon la revendication 1 ou la revendication 2, caractérisée en ce que :
   - l'angle d'inclinaison moyen du filetage rapide est au plus égal, et de préférence légèrement plus petit, que l'angle de frottement ($\mu$) des deux matériaux constituant les deux parties (44; 45) du filetage.

4. Soupape de purge selon une des revendications 1 à 3, caractérisée en ce que :
   - le palier d'appui (43) est constitué par une crapaudine conique creuse (42) et par une arête annulaire d'un tourillon de palier (29) qui est, de préférence, chanfreinée ou bien arrondie en section transversale,
   - de préférence la crapaudine (42) est disposée sur le bouchon fileté (37) et le tourillon de palier (29) sur le corps de soupape (25), auquel cas le tourillon de palier (29) est ramené à un peu plus petit diamètre que le corps de soupape (25).

5. Soupape de purge selon une des revendications 1 à 4, caractérisée en ce que :
   - le corps de soupape (25) est muni par rapport à sa surface d'étanchement, d'une dérivation (32) qui s'étend depuis la zone sans pression du corps de soupape (25) en direction de la zone sous pression jusqu'à un emplacement de sa surface d'étanchement qui, pour une course d'ouverture normale du corps de soupape (25) se trouve encore dans la zone de l'effet d'étranglement du siège de soupape (31),

cette dérivation étant constituée de préférence par un ou plusieurs évidements (32) qui, dans le cas où ils sont plusieurs, sont uniformément répartis sur la périphérie et sont, de préférence, orientés axialement.

Fig.1

Fig. 2